(19) Europäisches Patentamt
European Patent Office
Office européen des brevets

(11) Publication number: 0 066 224
B1

(12) EUROPEAN PATENT SPECIFICATION

(45) Date of publication of patent specification: 20.03.85

(51) Int. Cl.[4]: **C 07 C 47/12**, C 07 C 45/60, C 07 D 309/12, A 01 N 35/02

(21) Application number: 82104482.3

(22) Date of filing: 21.05.82

(54) **Preparation of a glutaraldehyde precursor.**

(30) Priority: 21.05.81 US 266102

(43) Date of publication of application:
08.12.82 Bulletin 82/49

(45) Publication of the grant of the patent:
20.03.85 Bulletin 85/12

(84) Designated Contracting States:
AT BE CH DE FR GB IT LI LU NL SE

(56) References cited:
FR-A-1 255 546
US-A-2 546 018
US-A-2 694 077
US-A-4 244 876

(73) Proprietor: UNION CARBIDE CORPORATION
Old Ridgebury Road
Danbury Connecticut 06817 (US)

(72) Inventor: Chu, Nan Shieh
7 Frost Lane
Hartsdale New York 10530 (US)

(74) Representative: Schmied-Kowarzik, Volker, Dr. et al
Patentanwälte Dr. V. Schmied-Kowarzik Dipl.-Ing. G. Dannenberg Dr. P. Weinhold Dr. D. Gudel Dipl.-Ing. S. Schubert Dr. P. Barz
Siegfriedstrasse 8
D-8000 München 40 (DE)

EP 0 066 224 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention is directed to a process for the preparation of a novel essentially nonaqueous glutaraldehyde precursor which is capable of rapidly generating glutaraldehyde upon addition to water. The precursor is prepared by the hydration of 2-alkoxy-3,4-dihydropyran in the presence of an acid catalyst.

Glutaraldehyde is widely used in many applications, such as leather tanning, embalming, photography and particularly as a microbiocide. Glutaraldehyde is generally prepared by the acid hydrolysis of 2-alkoxy-3,4-dihydropyran in a system containing a large excess of water. Without water, glutaraldehyde is generally not stable and polymerizes to a glassy-mass on standing. Thus, it is always prepared as an aqueous solution. A commercial concentration generally contains from about 50 to about 98 percent water. However, even this aqueous glutaraldehyde solution is capable of polymerizing to an oligomer and/or to a polymer which precipitates from the aqueous solution on standing.

Further, glutaraldehyde undergoes an alcohol condensation. The formation of these condensation products, as well as oligomers and/or polymers increases with, for example, increasing temperature, glutaraldehyde concentration, and pH.

The current processes for preparing glutaraldehyde have drawbacks. The formation of the condensation products and/or oligomers and polymers results in the loss of glutaraldehyde. Also, the use of 50 percent or more of water during the acid hydrolysis of the 2-alkoxy-3,4-dihydropyran decreases reactor capacity. Further, when the commercial 50 percent aqueous solution is shipped to warm climates, thermal stability of the solution becomes a serious problem. Also, in cold weather the aqueous solution may freeze.

Thus, there is much interest in developing a nonaqueous precursor to glutaraldehyde capable of yielding glutaraldehyde upon its addition to water. Such a precursor could be stored or shipped in varying climates without the resulting problems now associated with an aqueous solution.

US—A—4,244,876 describes the formation of a precursor to glutaraldehyde, i.e., 2,6-dimethoxytetrahydropyran. It is prepared by the addition of an alcohol to 2-methoxy-3,4-dihydropyran. However, the conditions under which the glutaraldehyde is produced from the 2,6-dimethoxytetrahydropyran are time consuming, since it requires several hours at low pH to generate a substantial amount of glutaraldehyde.

US—A—2,546,018 describes the preparation of glutaraldehyde by heating a derivative of dihydropyran, for example, a 2-alkoxy-3,4-dihydropyran with water. The principal product of the reaction is glutaraldehyde. Thus, the problems associated with aqueous or nonaqueous glutaraldehyde still exists.

In the present invention a novel essentially nonaqueous glutaraldehyde has been discovered.

This invention is directed to a process for the preparation of a novel essentially nonaqueous glutaraldehyde precursor. The precursor is prepared by hydrating 2-alkoxy-3,4-dihydropyran in the presence of an acid catalyst at a temperature of from 30 to 100°C and wherein the molar ratio of water to 2-alkoxy-3,4-dihydropyran is from 1:1 to 3:1. The hydration conditions are such that materials containing appreciable and substantial carbonyl content are not produced, i.e., essentially no free glutaraldehyde is produced.

The hydration product produced does not polymerize. Also, it has better thermal stability and a lower freezing point than a commercial 50 percent or greater glutaraldehyde solution. The hydration product yields glutaraldehyde upon addition to water.

The reaction may be illustrated by the hydration of, for example, 2-methoxy-3,4-dihydropyran:

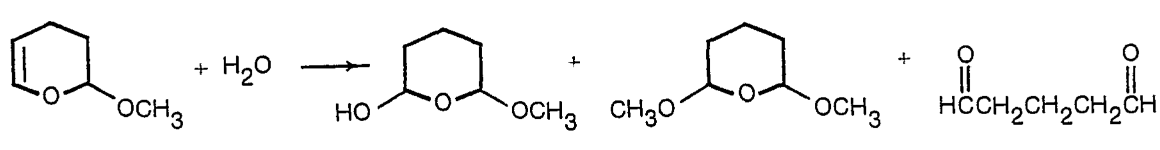

(I) (II) (III)

The principal product is 2-hydroxyl-6-methoxytetrahydropyran (I) although trace amounts of 2,6-dimethoxytetrahydropyran (II), glutaraldehyde (III) and their isomers are — or may be — formed.

The reaction is carried out in the presence of an acid catalyst. The acid catalyst may be an inorganic or an organic acid or ion exchange resin. The acids which may be used herein include a strong mineral acid, an acid-reacting salt, a material which will react under the conditions of the process to form in situ, an acid-reacting material or a supported acid catalyst. These acid catalysts include phosphoric acid, hydrochloric acid, sulfuric acid, trifluoromethylsulfonic acid, para-toluenesulfonic acid, as well as supported acid catalysts, such as Amberlyst® which is a supported arylsulfonic acid (sold by Rohm & Haas Company) and Nafion® which is a supported fluorosulfonic acid (sold by E. I. duPont de

Nemours Co.). When a liquid acid is used, the acid may be removed after hydration by neutralizing it with a base to form a salt and then removing the salt, as by filtration.

The reaction is carried out at a temperature of from 30 to 100°C, and preferably from 40 to 90°C. The reaction temperature and time are dependent upon the type and amount of catalyst used in the reaction.

The reaction is generally carried out at atmospheric pressure, although subatmospheric and superatmospheric pressures may be used.

The molar ratio of water to 2-alkoxy-3,4-dihydropyran in the reaction is from 1:1 to 3:1, and preferably from 1:1 to 2:1. The use of larger amounts of water offer no advantages since reactor capacity is reduced and the shelf-life of the reaction product is reduced.

The alkoxy group of the 2-alkoxy-3,4-dihydropyran contains from 1 to 3 carbon atoms.

The reaction product may be used as such. It is added to water to produce glutaraldehyde. The water may contain optional ingredients such as colorants, thickeners, and fragrances. However, water is the predominant material.

The following examples serve to give specific illustrations of the practice of this invention.

Example 1

A 250 ml 4-necked round bottom flask, fitted with a condenser, a mechanical stirrer, a thermometer and a nitrogen inlet was used as the reaction vessel. The flask was flushed with nitrogen and 114 g of 2-methoxy-3,4-dihydropyran, 21.6 g of distilled water and 13.2 g of Rexyn® 101, a sulfonated polystyrene (supplied by Fisher Scientific Co.) were added. The catalyst resin was rewashed with distilled water to remove most of the water soluble acids which might be present. The resin contained 5 milliequivalents of acid per gram of dry resin. The reaction mixture was kept in a constant temperature bath of 50°C. The hydration was followed gas chromatographically (Hewlett-Packard Model 5710A) to completion by the disappearance of the 2-methoxy-3,4-dihydropyran peak. The reaction was carried out for about 2 to 2-1/2 hours. The reaction was cooled to room temperature (about 25°C) and the reaction product filtered. A slightly yellow liquid was obtained. The yield was about 98 percent.

Examples 2 to 4

The procedure described in Example 1 was exactly repeated except that the molar ratio of water to 2-methoxy-3,4-dihydropyran was varied in Examples 2 to 4 as follows: 1.2/1.0; 2.0/1.0 and 5.6/1.0 for comparison. After the reaction was complete, portions of the reaction products were stored in an oven set at a temperature of 60°C for 27 days of thermal aging. 25 percent solutions of the aged samples in water were prepared and their UV absorbance (at 233 nm) were measured in a 0.1 mm silica cell (vs. air). (Beckman ACTA Model M VIII). The absorbences of the reaction product before and after thermal aging are listed in Table I. The 233 nm absorbance indicates the formation of undesirable aldol type condensation products.

TABLE I

| | | UV Absorbance | | |
|---|---|---|---|---|
| Example | Mole ratio of $H_2O$/MDP[1] when made | Unheated | After 27 days at 60°C | % increase |
| 2 | 1.2 | 0.751 | 1.001 | 33 |
| 3 | 2.0 | 0.454 | 0.650 | 43 |
| 4 | 5.6 | 0.347 | 1.414 | 307 |

[1]MDP=2-methoxy-3,4-dihydropyran

The data in Table I show that as the ratio of water to 2-methoxy-3,4-dihydropyran increases the solutions are less thermally stable.

Examples 5 to 7

These Examples demonstrate the rapid generation of glutaraldehyde when the reaction products produced by the hydration of 2-methoxy-3,4-dihydropyran are added to water at room temperature (about 25°C). The reaction products were prepared as described in Example 1 with the mole ratio of water to 2-methoxy-3,4-dihydropyran in Examples 5 to 7 as follows: 1.0/1.0; 1.5/1.0 and 2.0/1.0. Within five minutes after addition to water, gas chromatograph analyses was used to show the generation of the glutaraldehyde. These aqueous solutions were prepared to contain equivalent amounts of 2-methoxy-3,4-dihydropyran.

TABLE II

| Examples | Concentration of solution of the hydration product (ppm)[1] | Mole ratio of $H_2O$/MDP when made | Glutaraldehyde found (ppm)[1] |
|---|---|---|---|
| 5 | 132 | 1.0/1.0 | 67.2 |
| 6 | 136 | 1.5/1.0 | 73.3 |
| 7 | 150 | 2.0/1.0 | 80.0 |

[1](ppm)=parts per million.

Examples 8 to 10

These Examples demonstrate the heat stability of the reaction product produced by the hydration of 2-methoxy-3,4-dihydropyran prepared as in Example 1. The mole ratio of water to 2-methoxy-3,4-dihydropyran in Examples 8 to 10 was as follows: 1.2/1.0; 1.5/1.0 and 2.0/1.0. The reaction products were thermally aged for the time periods and at the temperatures shown in Table III. After thermally aging, the reaction products were added to water to reflect an equivalent initial pyran concentration. Gas chromatograph analysis was used to show the concentration of glutaraldehyde. The untreated samples was also analyzed. Control A is a 50 percent glutaraldehyde solution.

The results are shown in Table III.

TABLE III

| Example | Mole ratio of $H_2O$/MDP when made | Glutaraldehyde (ppm) | | |
|---|---|---|---|---|
| | | Untreated | After 9 days at 60°C | After 27 days at 60°C |
| 8 | 1.2/1.0 | 74.2 | 73.3 | 73.5 |
| 9 | 1.5/1.0 | 75.1 | 72.2 | 76.0 |
| 10 | 2.0/1.0 | 79.4 | 80.0 | 81.0 |
| Control A | 50% glutaraldehyde solution | 97.5 | 92.0 | 86.6 |

The data in Table III show that the reaction product of this invention is more thermally stable than the 50 percent aqueous glutaraldehyde solution.

Examples 11 to 14

These Examples demonstrate that the rapid generation of glutaraldehyde from the 2-hydroxy-6-methoxytetrahydropyran reaction product makes this product a very effective microbiocide. The products were prepared by the procedure described in Example 1. The mole ratio of water to 2-methoxy-3,4-dihydropyran in Examples 11 to 14 was as follows: 1.0/1.0; 1.2/1.0; 1.5/1.0 and 2.0/1.0. The concentration of reaction products required to kill all the *staphylococcus aureus* ($10^7$ colony forming units/milliliter) present with 2 hours contact time using a solution having a pH of 7 are shown in Table IV.

TABLE IV

| Example | Mole ratio of $H_2O$/MDP when made | Concentration of reaction product required (ppm as made) |
|---|---|---|
| 11 | 1.0 | 66 |
| 12 | 1.2 | 68 |
| 13 | 1.5 | 70 |
| 14 | 2.0 | 75 |

Example 15

The procedure described in Example 1 was exactly repeated except that the amounts of materials used were as follows: 28.5 g of 2-methoxy-3,4-dihydropyran, 5.4 g of distilled water and 6.6 g of catalyst. Also, the reaction was carried out at a temperature of 35°C instead of 50°C. The reaction was complete in about 2 hours.

Example 16

The procedure in Example 1 was exactly repeated except that the amounts of materials used were as follows: 28.5 g of 2-methoxy-3,4-dihydropyran and 4.5 g of distilled water. Also 1.0 g of Nafion-511 H® (0.95 milliequivalents per gram, supplied by E. I. DuPont de Nemours & Co.) was used instead of the Rexyn 101® catalyst. The reaction was complete in about 40 minutes.

Example 17

The procedure in Example 1 was exactly repeated except that the amounts of materials used were as follows: 28.5 g of 2-methoxy-3,4-dihydropyran and 5.4 g of distilled water. Also, 0.5 g of an 85 percent phosphoric acid solution was used at the catalyst instead of Rexyn 101®. The reaction was carried out at a temperature of 90°C instead of 50°C. The reaction was complete in about 90 minutes. The reaction product was cooled to room temperature (about 25°C) and adjusted to a pH of 7 with 0.36 g of sodium bicarbonate. The reaction product was filtered to remove a small amount of precipitate.

Example 18

The procedure of Example 17 was exactly repeated except that 1.0 g of the phosphoric acid solution was used. Also, the reaction was carried out at a temperature of 65°C. The reaction was complete in about 2 to 2-1/2 hours. The reaction product was cooled to room temperature (about 25°C) and adjusted to a pH of 7 with 0.72 g of sodium bicarbonate. The reaction product was filtered to remove a small amount of precipitate.

**Claims**

1. A process for producing a glutaraldehyde precursor characterized by hydrating 2-alkoxy-3,4-dihydropyran in the presence of an acid catalyst at a temperature of from 30 to 100°C, wherein the molar ratio of water to 2-alkoxy-3,4-dihydropyran is from 1:1 to 3:1 and wherein the alkoxy group contains from 1 to 3 carbon atoms.

2. A process as defined in claim 1 wherein the 2-alkoxy-3,4-dihydropyran is 2-methoxy-3,4-dihydropyran.

3. An essentially nonaqueous product comprising principally 2-hydroxy-6-methoxytetrahydropyran with trace amounts of 2,6-dimethoxytetrahydropyran and glutaraldehyde.

4. An essentially nonaqueous product comprising 2-hydroxy-6-methoxytetrahydropyran and 2,6-dimethoxytetrahydropyran.

5. 2-hydroxy-6-methoxytetrahydropyran.

6. A process for producing an aqueous solution of glutaraldehyde characterized by adding the reaction product of claim 1 to water.

7. A process for producing an aqueous solution of glutaraldehyde characterized by adding 2-hydroxy-6-methoxytetrahydropyran to water.

**Patentansprüche**

1. Verfahren zur Herstellung eines Glutaraldehydvorläufers, dadurch gekennzeichnet, daß man 2-Alkoxy-3,4-dihydropyran in Anwesenheit eines Säurekatalysators bei einer Temperatur von 30 bis 100°C hydratisiert, wobei das molare Verhältnis von Wasser zu 2-Alkoxy-3,4-dihydropyran von 1:1 bis 3:1 beträgt und wobei die Alkoxygruppe 1 bis 3 Kohlenstoffatome enthält.

2. Verfahren nach Anspruch 1, in welchem das 2-Alkoxy-3,4-dihydropyran 2-Methoxy-3,4-dihydropyran ist.

3. Ein im wesentlichen nicht-wässriges Produkt, das hauptsächlich 2-Hydroxy-6-methoxytetrahydropyran mit Spurenmengen von 2,6-Dimethoxytetrahydropyran und Glutaraldehyd enthält.

4. Ein im wesentlichen nicht-wässriges Produkt, das 2-Hydroxy-6-methoxytetrahydropyran und 2,6-Dimethoxytetrahydropyran enthält.

5. 2-Hydroxy-6-methoxytetrahydropyran.

6. Verfahren zur Herstellung einer wässrigen Glutaraldehydlösung, dadurch gekennzeichnet, daß man das Reaktionsprodukt von Anspruch 1 zu Wasser zugibt.

7. Verfahren zur Herstellung einer wässrigen Glutaraldehydlösung, dadurch gekennzeichnet, daß man 2-Hydroxy-6-methoxytetrahydropyran zu Wasser zugibt.

**Revendications**

1. Procédé de production d'un précurseur du glutaraldéhyde, caractérisé par l'hydratation d'un 2-alkoxy-3,4-dihydropyranne en présence d'un catalyseur acide à une température de 30 à 100°C, le rapport molaire de l'eau au 2-alkoxy-3,4-dihydropyranne étant d'environ 1:1 à 3:1 et le groupe alkoxy contenant 1 à 3 atomes de carbone.

2. Procédé suivant la revendication 1, dans lequel le 2-alkoxy-3,4-dihydropyranne est le 2 méthoxy-3,4-dihydropyranne.

3. Produit essentiellement non aqueux, comprenant principalement du 2-hydroxy-6-méthoxy-tétrahydropyranne avec des traces de 2,6-diméthoxytétrahydropyranne et de glutaraldéhyde.

4. Produit essentiellement non aqueux comprenant du 2-hydroxy-6-méthoxytétrahydropyranne et du 2,6-diméthoxytétrahydropyranne.

5. Le 2-hydroxy-6-méthoxytétrahydropyranne.

6. Procédé de production d'une solution aqueuse de glutaraldéhyde, caractérisé par l'addition à de l'eau du produit de réaction de la revendication 1.

7. Procédé de production d'une solution aqueuse de glutaraldéhyde, caractérisé par l'addition de 2-hydroxy-6-méthoxytétrahydropyranne à de l'eau.